(19) **Europäisches Patentamt European Patent Office Office européen des brevets**

(11) **EP 3 737 285 B1**

(12) **EUROPEAN PATENT SPECIFICATION**

(45) Date of publication and mention
of the grant of the patent:
**09.08.2023 Bulletin 2023/32**

(21) Application number: **19700171.2**

(22) Date of filing: **09.01.2019**

(51) International Patent Classification (IPC):
*A61B 5/107* (2006.01)      *A61B 1/00* (2006.01)
*A61B 1/06* (2006.01)       *A61B 1/05* (2006.01)
*A61B 5/00* (2006.01)       *G01B 11/25* (2006.01)

(52) Cooperative Patent Classification (CPC):
**G01B 11/2509; A61B 1/000094; A61B 1/00096;
A61B 1/00194; A61B 1/05; A61B 1/0605;
A61B 1/0638; A61B 5/1076; A61B 5/1079;**
A61B 1/0607; A61B 5/0084

(86) International application number:
**PCT/EP2019/050418**

(87) International publication number:
**WO 2019/137946 (18.07.2019 Gazette 2019/29)**

(54) **ENDOSCOPIC NON-CONTACT MEASUREMENT DEVICE**

ENDOSKOPISCHE VORRICHTUNG ZUR KONTAKTLOSEN MESSUNG

DISPOSITIF DE MESURE ENDOSCOPIQUE SANS CONTACT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.01.2018 EP 18151091**

(43) Date of publication of application:
**18.11.2020 Bulletin 2020/47**

(73) Proprietor: **Université Libre de Bruxelles
1050 Bruxelles (BE)**

(72) Inventors:
• **MERTENS, Benjamin
  1332 Genval (BE)**
• **DELCHAMBRE, Alain
  1170 Bruxelles (BE)**

• **PETRE, Maxime
  1180 Bruxelles (BE)**
• **DEVIERE, Jacques
  1404 Bornival (BE)**
• **LEDUC, Dimitri
  1200 Bruxelles (BE)**

(74) Representative: **AWA Benelux
AWA Benelux SA
Tour & Taxis - Royal Depot box:216
Havenlaan 86c Avenue du Port
1000 Bruxelles (BE)**

(56) References cited:
**WO-A2-2017/125926      US-A1- 2012 190 923
US-A1- 2016 143 509      US-A1- 2016 302 653
US-A1- 2017 168 232**

## Description

## Technical field

The present invention is related to devices and methods for non-contact measurement, in particular internal cavity scoping devices and methods, such as, but not limited to, endoscopic devices and methods, which allow to perform noncontact dimensional measurements on a surface of the internal cavity. The non-contact measurement is advantageously performed by triangulation to obtain a measurement, e.g. of a distance between two points, such as a cavity diameter.

## Background art

In the medical field, airway measurement is receiving increased interest, notably prior to stent placement. Physicians need to measure the bronchi diameter in order to select the appropriate stent or to measure airway stenosis. For these measurements, physicians currently use either the CT-Scan, which irradiates the patient or a biopsy forceps used as a size reference, but which is not so accurate as a measurement tool. The diameter of an airway can vary within a relatively large range: more than 20 mm for the trachea down to almost 2 mm for the smallest bronchi.

From US 2012/0190923 an endoscope is known which measures the topography of a surface. The endoscope contains a projection unit and an imaging unit. The projection unit and the imaging unit are arranged successively in relation to an axis of the endoscope. The configuration of the projection unit and the imaging unit arranged axially behind one another on the axis permits a significantly smaller endoscope configuration. The endoscope comprises a projection structure in the form of a slide having a radially symmetrical structure. The projection structure is illuminated by a waveguide to obtain a colour coded light pattern. One drawback of the above device is that its light pattern projector has a fairly complex structure with moving parts (the slider), which prevents the endoscope to be further miniaturized. Another drawback of the above device is that it requires a user handling a user interface when simple measurements, such as size or diameter are required. This can be cumbersome, since the operator of the device will generally have both hands involved in guiding and/or positioning the endoscope.

WO 2017/125926 discloses an intraoral scanner including a light projector which is a coherent light generator that generates a diffractive light pattern on a region of interest, the light pattern including at least two different wavelengths. The intraoral scanner can comprise a diffractive optical element (DOE) that creates a given pattern at two or more wavelengths such that the patterns differ by the wavelengths ratio. One embodiment of the intraoral scanner includes a laser source which generates three laser beams at RGB wavelengths via a single optical fiber to DOE 1204 that casts a pattern on a region of interest.

US 2016/0143509 discloses a system for stereo reconstruction comprising a light patterning component configured to be disposed within the working channel of a monoscopic endoscope such that a light emitting end of the light patterning component will be fixed with a defined relative distance from the distal end of an image pick-up element. The light patterning component forms a pattern of light that is projected onto a region of interest. A distance from the endoscope to the region of interest is determined based on the image signal and based on the defined relative distance between the light emitting end of the light patterning component and the distal end of the image pick-up element.

## Summary of the invention

It is an object of the invention to provide an endoscopic non-contact measurement device which can accurately measure internal cavity diameters of a large size range, and which would particularly be useful in human airways. It is an object to provide endoscopic non-contact measurement devices that can be miniaturized for use in instrument channels of endoscopes, such as channels having a diameter of 2.8 mm or less, in particular instrument channels of bronchoscopes.

It is an object of the invention to provide an endoscopic non-contact measurement device which is of simpler structure and/or which is easier to use. In particular, it is an object to provide such devices which allow for taking automatic measurements in real time, preferably without any user intervention.

According to the invention, there is therefore provided a device for non-contact measurement as set out in the appended claims. Devices according to the present invention are advantageously configured to perform a measurement at a target site remotely, i.e. without need to contact measurement points at the target site. The device comprises a light source and a light pattern projector comprising a diffractive optical element optically coupled to the light source, an imaging system configured for imaging a target site illuminated by the light pattern projector, a support to which the light pattern projector and the imaging system are attached in fixed relative positions, and a processing unit configured to process data acquired by the imaging system.

The support has a longitudinal axis parallel to an optical axis of the light pattern projector, wherein the light pattern projector and the imaging system are arranged at spaced apart positions along the longitudinal axis. The light pattern projector, in particular, the diffractive optical element, and the imaging system are advantageously arranged the one behind the other. They are advantageously arranged coaxially. The light pattern projector is advantageously configured to project a pattern having a (substantially) rotational symmetry about the optical axis.

According to the present invention, the light source is operable to emit a plurality of light beams of different colours. The processing unit is configured to control the light source and/or the light pattern projector such that a single one of the separate patterns is projected at an instant of time, and/or the device comprises a user interface allowing for selecting a single one of the separate patterns for projection. Each one of the plurality of light beams is a coherent beam. The diffractive optical element, which is optically coupled to each one of the coherent beams, is configured to diffract the plurality of light beams according to separate or distinct patterns. The separate patterns result from the diffractive optical element diffracting the plurality of light beams having a different colour according to different angles due to the different wavelengths of light.

According to the invention, the processing unit is configured to determine a measurement based on at least two positions detected in data acquired from a single one of the separate patterns. The at least two positions are automatically recognised by the controller in the acquired data, e.g. through a suitable image recognition algorithm. In other words, only one pattern of a single one of the different colours is used to perform a measurement at a time.

An advantage of the above aspects is that the indicated device can be used as pointer device for making automatic measurements. By way of example, the surgeon or operator points at least one of the separate patterns at the target location, where measurement is desired, e.g. on a bronchus in a human airway, on a restriction in a lumen, etc., and the device will automatically perform a desired measurement at the location that is pointed at with the light pattern, e.g. of the size of the bronchus, or the diameter of the restriction. The measurement can be performed automatically, advantageously because it is based on only one of the separate patterns. The measurement is therefore made with the operator only needing to correctly point the device, as can be verified e.g. on a visual display, without any further intervention. Devices according to the invention therefore allow for faster diagnosis and imaging.

It will yet be easier for the processing unit and for the operator, when the projected pattern is of simple shape, and allows for correct visual positioning, e.g. a pattern having rotational symmetry, advantageously a single circle, or a plurality of points or line segments arranged on a (single) circle. The measurement can be a diameter or perimeter of the (circle) pattern, or an area (surface) enclosed by the circle pattern. There is hence a one to one registration between the measurement and the pattern, which makes it easy for the operator to correctly position or point the device on the measurement points.

Advantageously, the processing unit is configured to process each of the separate patterns individually. Still advantageously, the processing unit is configured to determine for each (or multiple ones) of the separate patterns a corresponding measurement. Each of these measurements is advantageously based on at least two positions detected in data acquired from the respective one of the separate patterns only. While it is known that different wavelengths of light will be diffracted under different angles, in the present aspect this is exploited to create separate projection patterns. The separate patterns are advantageously spaced apart. The separate patterns, each one relating to a different one of the plurality of light beams, advantageously have different sizes when projected on a same plane. The inventors have shown that measurements at a same target distance from the device and made based on patterns of different colour (i.e., different light wavelengths) will have different accuracy considering a same distance between the measurement device and the target site. It has been shown that measurements with patterns formed by a light beam of larger wavelength (e.g. red colour) have better accuracy than when a pattern formed by a light beam of shorter wavelength (e.g. blue colour) is used. Larger wavelength beams will however be diffracted according to larger angles and therefore form patterns of larger size. Such larger patterns may not cover smaller features at the target site. Therefore, depending on the size of the feature that one wants to measure, devices according to present aspects allow for using patterns of different light beam wavelengths, so that a same relative accuracy is obtained for measurements of any size. As a result, the measurement accuracy is guaranteed for any lumen sizes or depths within the measurement range.

Advantageously, the processing unit can be coupled to the light source for controlling which one of the plurality of light beams, or which one of the separate patterns, to emit. Advantageously, the pattern or light colour can be automatically selected, e.g. by the processing unit. It will be convenient to note that the separate patterns indicated above are distinct patterns and not colour coded patterns. Therefore, colour coded patterns are advantageously not used in aspects of the present invention.

According to a second aspect of the present disclosure not forming part of the invention, there is provided a method of performing a non-contact measurement. The method comprises projecting a plurality of separate patterns on a target site in the internal cavity. The plurality of patterns originate from a plurality of light beams of different colours. Each one of the plurality of light beams is a coherent beam. The plurality of light beams are diffracted to separate ones of the plurality of patterns. The plurality of patterns is imaged and a measurement is determined based on at least two positions of a single one of the separate patterns, e.g. via triangulation. Advantageously, a measurement can be determined for each of the separate patterns, e.g. based on at least two positions of each of the patterns. The plurality of separate patterns are advantageously not projected simultaneously, and a user may select, e.g. through a user interface, which one of the plurality of separate patterns to project. Advantageously, the measurement is determined by automatic recognition of measurement points at the target site. Present methods are advantageously performed using devices according to the present invention.

**Brief description of the figures**

Aspects of the invention will now be described in more detail with reference to the appended drawings, wherein same reference numerals illustrate same features and wherein:

Figure 1 represents various components of a device according to aspects described herein;
Figure 2 represents an optical assembly of the distal part of a device according to some aspects described herein;
Figure 3 represents a mathematical model of the optical assembly of Fig. 2;
Figure 4 represents a schematic setup of a device according to aspects described herein;
Figure 5 represents a graph showing the absolute measurement error in relation to the measurement depth for light patterns of different wavelength (blue, green and red), pixel error = 0.5 pixel, focal length = 165 pixels, baseline = 5 mm;
Figure 6 represents a graph showing the absolute measurement error of the diameter in relation to the measured diameter for light patterns of different wavelength (blue, green and red), pixel error = 0.5 pixel, focal length = 165 pixels, baseline = 5 mm;
Figure 7 represents a probe of a device according to a further aspect described herein;
Figure 8 represents a probe of a device according to a yet further aspect described herein;
Figure 9 represents a probe of a device to which an external instrument channel is attached, for guiding the probe there through according to a further aspect described herein;
Figure 10 represents a set of separate patterns projected on a wall obtained by emitting light beams of three different wavelengths through a same diffractive optical element.

**Description of embodiments**

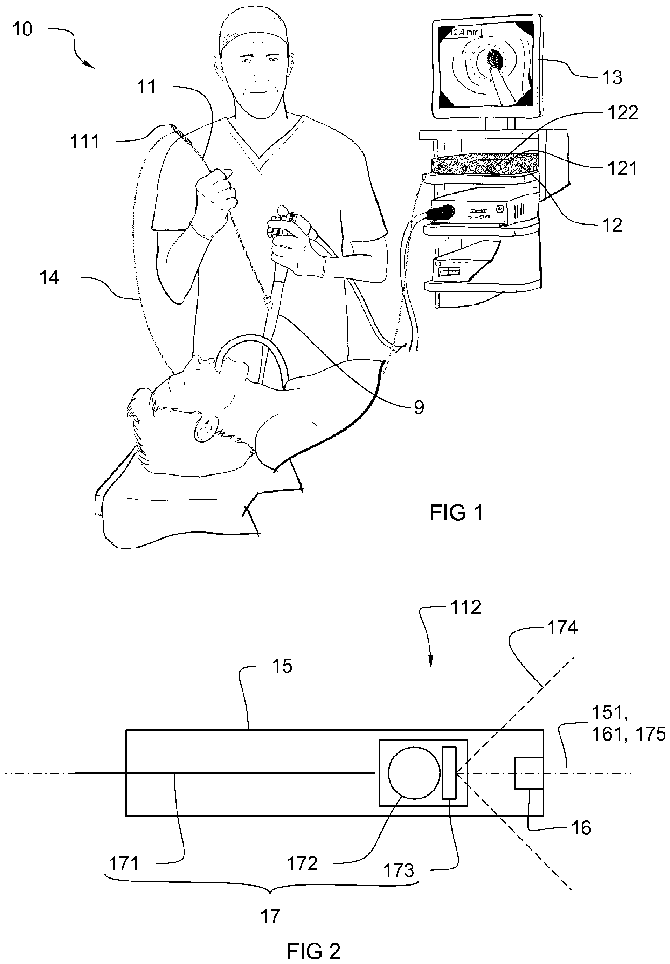

Referring to Fig. 1, non-contact measurement device 10 generally comprises a probe 11 connected to a processing unit/controller 12 through a cable 14. It is alternatively possible to couple probe 11 wirelessly to controller 12. In the instant example for use in medical imaging, the probe 11 is configured to be inserted in the patient, either through the instrument channel of an endoscope 9 or through any other guiding endoscopic tools, such as a trocar. Probe 11 may alternatively be inserted directly in the patient (e.g. it may be formed as an endoscope) without requiring additional guiding tools. The controller 12 can be connected to a visual display 13 and be configured to display results on the display.

The probe 11 may be formed as a flexible and elongate device, extending between a proximal end 111 to which cable 14 is connected, and a distal end 112 which forms the end which is inserted first in the patient and therefore the end which is most remote from the operator. Referring to Fig. 2, at the distal end 112 of probe 11, device 10 comprises a support to which an imaging system 16 and a light pattern projector 17 are attached in a fixed and advantageously spaced apart relationship. The support is advantageously arranged as a housing 15 which may be elongate and advantageously cylindrical. The support may be made of a transparent plastic, such as PMMA, polycarbonate, or glass. It can be either moulded or machined. Advantageously, the probe is small enough to travel through instrument channels of endoscopes. Specific examples of instrument channel diameters are 2.8 mm, 2.1 mm and 2 mm.

The imaging system 16 is configured for capturing an advantageously pixelized image of the light pattern projected by the light pattern projector 17. To this end, imaging system 16 can comprise an imaging sensor, such as a CCD or CMOS sensor, possibly coupled with an optical lens. The imaging system is connected to controller/processing unit 12 and possibly to a power supply located at the proximal end, e.g. within controller 12, through power and/or signal transmission cables. Alternatively, imaging system 16 can comprise an independent (carry-on) power supply, such as a battery. A suitable example of imaging sensor is the Omnivision® OV6946A, which may allow the device to be used for general inspection, such as inspection of bronchi. The device 10 can comprise a light source which is advantageously arranged remotely, e.g. in controller 12. The light source can alternatively be arranged within housing 15. An optical fibre 171 advantageously connects the light source to the light pattern projector 17, which typically is arranged at the distal end 112, within and advantageously fixed to the housing 15. The light pattern projector comprises a diffractive optical element (DOE) 173, such as DE-R 220 provided by Holoeye Photonics (Germany). The optical system may further comprise one or more lenses 172 for collimating the light beam carried by the optical fibre 171. A specific example of a collimating lens is a ball lens, such as the 2.0 mm diameter Fused Silica Ball Lens from Thorlabs (USA).

In the instant example, the imaging system 16 is arranged in front of the optical system of light pattern projector 17 in order to have a large field of view, and both are advantageously aligned on a longitudinal axis 151 of the housing 15. By so doing, the imaging system 16 is located within the field of illumination 174 of the light pattern projector 17. The imaging system 16 is however spaced apart from the (optical system of the) light pattern projector 17 along the longitudinal axis 151, and therefore the imaging system 16 only partially obstructs the field of illumination 174 of the light pattern projector 17. It is alternatively possible to interchange the positions of the imaging system 16 and the light pattern projector 17. In other words, the light pattern projector 17 can be placed in front of the imaging system 16.

The optical axis 161 of the imaging system 16 is advantageously coincident with the optical axis 175 of the light pattern projector 17 and either one or both the optical axes 161, 175 can be coincident with the longitudinal axis 151. Alternatively, optical axis 161 can be offset from optical axis 175, e.g. optical axes 161 and 175 can be substantially parallel and spaced apart. Optical axis 175 can e.g. correspond to an optical axis of the DOE 173.

Due to the placement of imaging system 16 in front of the light pattern projector 17, measurements can be performed of the target site which is illuminated by light pattern projector 17 and which is not shadowed by the imaging system 16. Imaging system 16 can be provided with a suitable lens adapted for imaging at least part of the field of illumination 174. Triangulation techniques can be applied on that part of the target site illuminated by the light pattern and imaged by system 16 since projector 17 and imaging system 16 are spaced apart.

One advantage of the disposition of Fig. 2, is that the distance between the light pattern projector 17, in particular the optical system and more particularly the DOE 173, and the imaging system 16 can advantageously be selected as desired without increasing the external diameter of the housing 15. As a result, the probe 11 can be made extremely compact to decrease the bulkiness of the system, yet providing desirable measurement accuracy. With such a configuration, probe 11 can be made sufficiently small allowing for insertion through an instrument channel or lumen of an endoscope. The probe 11 is therefore advantageously able to travel along with the endoscope to reach the target site. In the alternative, it is possible to provide probe 11 with a suitable connector, for external attachment to the endoscope. Also in this case, bulkiness is reduced.

The above arrangement of light pattern projector and imaging system (Fig. 2) advantageously uses a circular or at least rotationally symmetrical pattern, e.g. containing a unique point around the optical axis, such as a circle, a square, two parallel lines,...that is projected. In this case, deformation of the pattern occurs radially. To this end, the optical system and the DOE in particular advantageously have optical properties that have rotational symmetry all about the optical axis 175.

The projected pattern is preferably circular, or advantageously has rotational symmetry about the optical axis 175. The projected pattern may be formed of dots, e.g. arranged in a circle, or even a line, circle segment, arc, or any other suitable pattern which allows analysis by computer. Advantageously, the light pattern is one which is not visually invasive and advantageously does not impede viewing of the internal cavity, e.g. by an endoscope monitor. The light pattern is advantageously of simple shape, e.g. a line, circle, or a plurality of dots or markers. The light pattern, such as the ones above, advantageously allows the user to position the pattern on the spot at which a measurement is desired. In particular, devices as described herein are configured for measuring distance, such as lengths (e.g., perimeter) or diameters. To this end, the light pattern should be positioned on the at least two points on target site between which distance is to be determined. Therefore, the light pattern is advantageously such that it easily allows for being positioned on two or possibly more points at the target site simultaneously. Patterns which depart from the circular geometry may be used as well, e.g. patterns with rotational symmetry over given angles, such as squares and other polygons.

As the light pattern is imaged by the imaging system 16, its image is acquired by the controller 12, which comprises processing means for automatic recognition of the light pattern, and for determining distance, advantageously in real-time. Generally, the more the light pattern will be of simple geometry, the easier the processing will be.

The light source coupled to the light pattern projector is advantageously a monochromatic narrowband light source, typically a laser diode. The light source is advantageously operable to emit coherent light. One suitable example of such a light source is LP450-SF15from Thorlabs, USA. The light source can be arranged in controller 12. The optical fibre 171 is then used to carry the light beam emitted by the light source to the light pattern projector 17 in housing 15. A suitable example of optical fibre is S405-XP from Thorlabs, USA. Alternatively, the light source can be provided in housing 15.

Advantageously, the pattern assists the user in defining the measurement point(s) and/or enables automatic recognition of the measurement point(s), such as e.g. two points between which a diameter or distance is desired. It is thus desirable that the light source is within the visible range, in order to make the light pattern easy to be seen by the physician which is guiding the probe 11 and/or the endoscope 9. An automatic recognition advantageously enables making automatic measurements without any user handling on a user interface, such as a graphical user interface. Referring again to Fig. 1, a measurement can be shown on the visual display 13 without the user having to select points with a pointing device. It will be convenient to note that the light pattern is dedicated for making distance measurements, such as a measurement between two points on the target site, for determining e.g. lumen size, diameter, width, etc., or area measurements, and is not primarily intended for surface topography. Surface topography is advantageously not implemented in devices according to the invention. The light pattern advantageously allows for making real-time distance measurements. Visualization of the measured distance advantageously assists the user simultaneously with imaging of the anatomical structure, e.g. as performed by the endoscope camera, and with making a correct diagnose with no risk of error due to bad 3D reconstruction. Another advantage is that the same arrangement can be used to project different pattern shapes simply by changing the DOE.

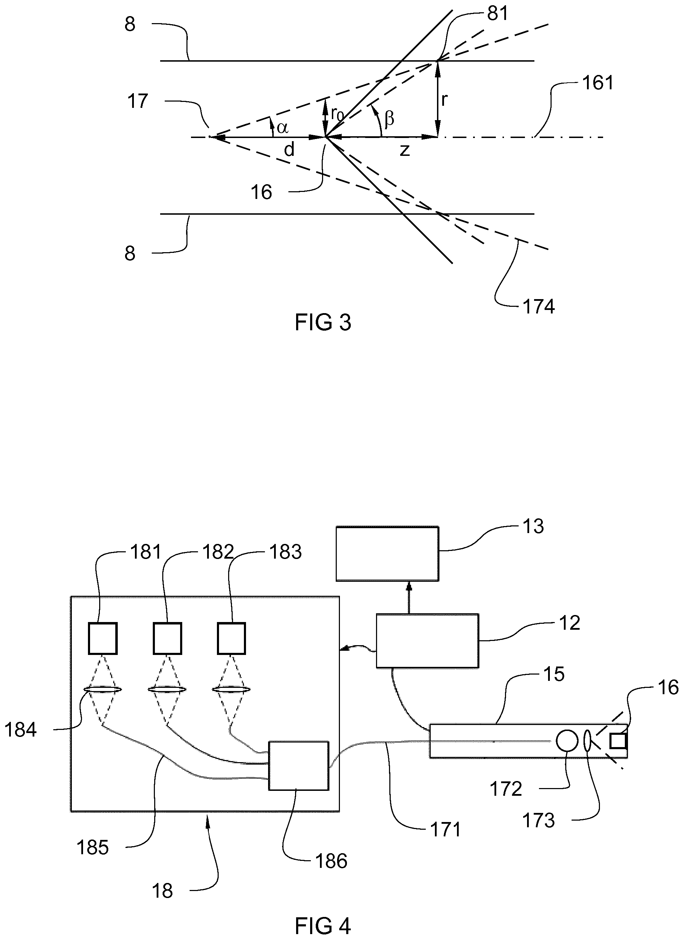

Referring to Fig. 4, in one aspect the light source is operable to emit a plurality of coherent light beams. The coherent light beams are of different colours, e.g. they form a set of primary colours such as though not limited to blue, green and red (RGB system, even though other systems may be used as well). To this end, the light source 18 can comprise a plurality of coherent light sources 181, 182, 183, e.g. different LEDs, each one emitting a different colour (light wavelength). Each of the plurality of light sources is advantageously coupled to the same DOE 173. Each coherent light source 181-183 may comprise suitable optics, such as a collimating lens 184 to collimate the light beam before it is fed to an optical fibre 185 which may carry the light beam to the DOE 173 at the distal end. Since the diffraction angle at the DOE 173 depends on the wavelength, one advantage of using coherent light beams is that each of the coherent light beams will be diffracted by a same DOE 173 with a different projection (diffraction) angle. By way of example, a DE-220 diffractive optical element (Holoeye, Berlin), will project a light beam of 450 nm at an angle of 19°, a light beam of 532 nm at 23° and a light beam of 650 nm at 28°. At a distance of 100 mm from the light pattern projector, the symmetrical pattern formed by the DE-220 DOE and obtained with the 450 nm light beam will have a size (diameter) of 70 mm, the pattern obtained with the 532 nm light beam will have a size of 85 mm and the pattern of the 650 nm light beam will have a size of 110 mm.

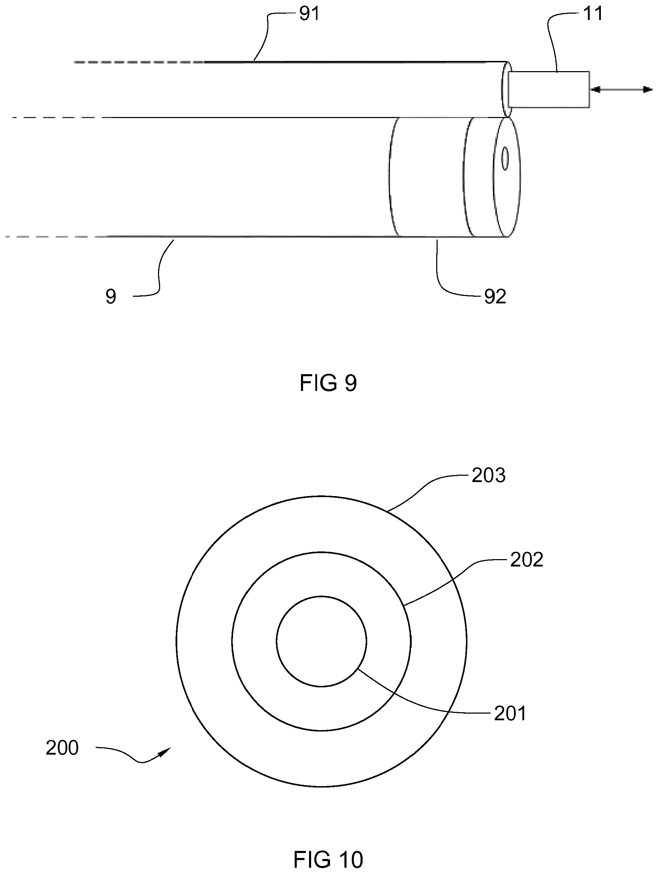

Fig. 10 shows one example of a set 200 of separate concentric patterns as can be obtained in case of a circle as pattern. Innermost pattern 201 is a pattern obtained by a light beam of a colour having smallest projection angle (e.g. 450 nm in the above example). Outermost pattern 203 is obtained by a light beam of a colour having largest projection angle (e.g. 650 nm in the above example), and intermediate pattern 202 is e.g. obtained with the 532 nm light in the example above. It can be seen that the smaller pattern is advantageously completely contained in the larger pattern. Needless to say, other kinds of patterns are possible as well. Therefore, the coherent light beams will form spaced apart light patterns at the target site. These light patterns may have a same shape, but will have different sizes, e.g. in case of circles, each colour light beam will project as a circle of different diameter at the target site due to differing projection (diffraction) angles. The circles are advantageously concentric. A concentric pattern generation has the advantage that it requires no additional space. The smaller the wavelength, the smaller the pattern. The use of several concentric patterns of different colours has the advantage that the pattern which best fits the lumen size being studied can be easily chosen for measurement. At the target site, a user may choose between different coherent light beams the one which best fits the cavity feature that is to be measured.

Therefore, measurements made with devices of aspects described herein advantageously rely on only one of the plurality of separate patterns, i.e. they rely on a pattern obtained with only one colour wavelength. At least two, possibly more positions at the target site may be determined based on data acquired from a single one of the plurality of patterns. The controller is advantageously configured to determine a measurement, such as a diameter, based on these positions only. The above may be repeated for each of the separate patterns, i.e. for each of the plurality of light beams. Each measurement made by present devices therefore is advantageously in one to one registration with one light beam colour. As a result, measurements are obtained which may relate to a same feature at the target site and which may have different accuracy as these measurements are obtained through the different patterns.

In one alternative, the light beams of different colours are applied consecutively to the probe 11. In another alternative not part of the present invention, a beam combiner 186 is coupled to the plurality of coherent light sources 181-183 for combining the coherent light beams into a single waveguide, e.g. the optical fibre 171. This allows for simultaneous projection of light patterns corresponding to the different colour light beams.

Referring back to Fig. 1, a user interface 121 can be provided, coupled to controller 12. The user interface is advantageously configured to allow the operator to select which one of the different colour light beams to project. By way of example, the user interface 121 can comprise a control knob 122 to this end. The specific choice of pattern can depend on the actual size of the structure that is to be measured and/or on the desired accuracy.

Referring to Fig 3, an example is shown of measurement of the wall 8 of an internal channel, such as an airway channel, e.g. the wall of a bronchus. The light pattern projector 17 has a projection angle α (for a given wavelength), the imaging system 16 has a Field-of-view (FOV), defined by its focal length $fx$ (in pixel) and the pattern is detected on the wall 8 at the target location 81 with an angle β. The distance between the imaging system and the light pattern projector is $d$ and the measurement depth (distance between imaging system and measurement point 81, along the optical axis 161) is $z$. The radius of the channel is $r$ and $r0$ is the radius of the light pattern FOV at position of the imaging system 16. It can easily be shown that:

$$\tan \beta = \frac{p_x}{f_x}.$$

Depth (z) is given by:

$$z = \frac{d \tan \alpha}{\tan \beta - \tan \alpha} = \frac{r_0}{\tan \beta - \tan \alpha}.$$

In the above, $p_x$ is the pixel position of the detected point. The above equations are given for a given plane, and are valid for every plane around the longitudinal axis.

In the following it is shown that the measurement accuracy for a given projection angle $\alpha$ depends on the size of the channel (radius $r$). Depth error $\delta z$ is given by:

$$\delta z = \frac{\delta p_x \, z^2}{f_x \, d \tan \alpha} = \frac{\delta p_x \, z^2}{f_x \, r_0}.$$

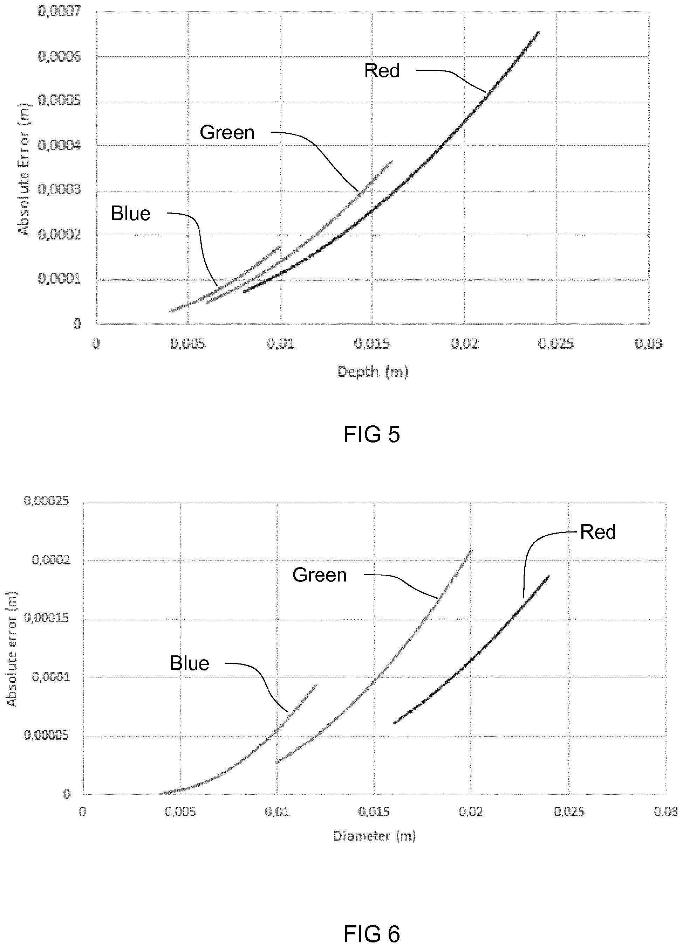

Figure 5 shows a plot of the error $\delta z$ in function of depth $z$. It represents the measurement accuracy of the device in relation to depth for three different light wavelengths and using a DOE DE-R 220 (Holoeye, Berlin).

For a diameter measurement, depth is less important. It can be shown that the error $\delta r$ on radius ($r$) is given by:

$$\delta r = \frac{\delta p_x \, (r - d \, \tan \alpha)^2}{f_x \, d \tan^2 \alpha} = \frac{\delta p_x \, (r - r_0)^2}{f_x \, r_0 \, \tan \alpha}.$$

The error $\delta r$ on the radius is plotted in Fig. 6 as error on diameter in relation to the lumen diameter. Both Fig. 5 and Fig. 6 show that sub-mm accuracy is achievable. In order to increase the accuracy, light of a larger wavelength can be used. However, this results in larger patterns which may not be visible in small lumens, since there may be no overlap between the imaging area (camera FOV) and the projected pattern at the target site. In small lumens (or for measuring small features), it is advantageous to use a small pattern to make the measurement. As shown in Fig. 6: for a given diameter, the absolute error decreases with increasing wavelength. For a given wavelength, the absolute error increases exponentially with diameter. From the plots it can be deduced that larger wavelengths will give a more accurate measurement for larger diameters. Smaller wavelengths are advantageously used for smaller diameters, in particular because larger wavelengths will be diffracted with larger angles and the resulting pattern may be too large and fall out of range of the boundary of the target structure. In the particular example of determining the size of a bronchus, a pattern formed with red coloured light (larger wavelength) may be too large and therefore may be hidden from the camera field of view in the bronchus. In such case, either the operator, or the system itself may switch to a pattern of different colour, e.g. of blue light (smaller wavelength) resulting in a smaller pattern which may be visible by the camera. In case the bronchus size is large, both the red light and blue light patterns will be visible. However, measurements made based on the red light pattern will have better accuracy. Therefore, the device may be set to use the red light pattern in this case.

It is alternatively possible, if space requirements permit, to position the light pattern projector 17 and the imaging system 16 in a juxtaposed and spaced apart position, instead of aligning them on a same axis. In such case, it may be advantageous to project linear patterns, e.g. straight lines, or a plurality of dots or markers aligned along straight lines. The DOE 173 will diffract different wavelengths, such as red, green and blue light with different projection angles, and the spacing between parallel pattern lines will differ between different light wavelengths. Such an arrangement may be useful e.g. for measuring vocal folds.

It is known that an imaging sensor (CCD or CMOS) is typically a single chip with a planar matrix of light sensitive elements. The energy of the light incident to each element is converted into a signal charge which is output from the sensor. This charge, however, only represents the intensity of the light that was incident on a particular light sensitive element. It does not produce colour images. To produce colour images, in general, digital imaging systems employ a filtering scheme to look at the incoming light in a bundle of primary colours, typically three, such as red, green and blue (RGB). There are basically two possible ways of organising a digital imaging system to produce colour images. In a first possibility, each of the light sensitive elements has broadband spectral sensitivity. A cooperating filter disc passes a series of colour filters, e.g., red, green and blue filters, through the light beam in a repeating time sequence. The filter interpositions are synchronised to image scanning, with the filter typically being interposed during an entire field scan. Devices operating in this manner are said to produce a "field sequential" colour signal. The filter disc can be arranged either at the light projector side, e.g., before light is emitted to the target site, or at the imaging system side, e.g. prior to the light being incident on the light sensitive elements. In an alternative possibility, a mosaic of individual selectively transmissive filters is superposed in one-to-one registration with individual light sensitive elements. Neighbouring light sensitive elements have superposed a filter which selectively transmits a different primary colour. The signal charges acquired by these neighbouring light sensitive elements are therefore representative of different primary colours. The image is then digitally reconstructed by interpolating the colour for each pixel of the image using the intensity of the colours detected at elements in a neighbourhood around the pixel location. Such interpolating algorithms are referred to as demosaicing algorithms. One known type of such mosaic filter is a Bayer filter and described in further detail in US 3971065 to Bayer, 20 July 1976.

Regardless of the filtering scheme employed in a digital imaging system, the imaging sensor advantageously acquires signal charges relating to the different spectral regions of the light reflected by the target site separately, such as though not necessarily in different channels, e.g. a red channel, a green channel and a blue channel. The different channels are coupled for acquisition to the processing unit 12 which can be implemented with a demosaicing algorithm to produce a desired colour image.

In one aspect of the invention, the coherent light beams projected by the light pattern projector 17 are individually acquired in different signals, e.g. through different channels of the imaging system 16. By way of example, coherent light sources 181-183 are configured to emit light in wavelength regions corresponding to those of the different channels of the imaging sensor, e.g. the primary colours such as red, green and blue. The processing unit 12 is advantageously implemented to process the colour signals corresponding to each coherent light beam emitted by the light pattern projector 17 separately from one another. By so doing, image segmentation for detecting the different light patterns is greatly simplified. Light pattern reconstruction is consequently more robust resulting in a more reliable measurement.

It will be convenient to note that other suitable colour (spectral) schemes can be applied, even employing more than three channels. Additionally, applicable filtering schemes are not limited to visible light. By way of example, the light pattern projector 17 can be configured to emit light in the infrared spectral region, particularly in a near infrared spectral band. Advantageously, each of the light patterns reflected by the target site 8 is acquired as a separate (colour) signal, with no or insignificant interference from light emitted by the other light beams.

In this regard, it is not required that the spectral region of a coherent light beam be captured by only one channel of the imaging sensor. By way of example, a yellow pattern (588 nm) is captured simultaneously by the green and red channel of a RGB imaging sensor and is not present on the blue channel.

The controller 12 is advantageously configured to process the data acquired by the imaging system 16. As already indicated, the sensor of the imaging system 16 may comprise different colour channels, and data from each such colour channel may be acquired separately by the controller 12. The controller 12 may be configured to process the data of each colour channel separately. Multiple colour channels may each acquire a light pattern relating to a different colour. These light patterns may be distinct, i.e. each pattern is created by a different light wavelength of the light pattern projector 17 / light source 18. Alternatively, or in addition, some (but not all) the colour channels may acquire data relating to a same light pattern, e.g. where the light pattern is projected in a light wavelength which is captured by two (or possibly more) colour channels of the imaging system sensor. The controller 12 may be implemented with a suitable algorithm for detecting the light patterns. By way of example, a background subtraction method, e.g. as available from the OpenCV library, could be used to easily detect patterns. In case of RGB colour channels, and a light pattern with a yellow light wavelength being used, the red and green channel can be merged and subtracted from the blue channel.

The controller 12 may be configured to determine a (distance) measurement between two points at the target site for at least one, and advantageously for each of the light patterns acquired, or alternatively, for each of the colour channels that it acquires. These different measurements advantageously relate to one and the same feature at the target site. On the basis of the distances or measurements that have been determined, the controller 12 may be configured to determine the distance with lowest measurement error. As indicated above, since the measurement error for a given distance is dependent on the wavelength of the light pattern, the controller can easily verify which of the wavelengths used will result in highest accuracy. This may be accomplished e.g. by storing a lookup table in readable memory included in the controller 12. The lookup table may comprise for each wavelength, a measurement error related to a given distance measurement. In this case, controller 12 may be configured to compare entries of the lookup table for a given distance.

The controller 12 may be configured to determine three dimensional coordinates of points along the imaged pattern. In case of rotationally symmetrical patterns, such as a circle, with the coordinates of circle points, the controller may be configured to calculate one or more diameters, such as one or more of: an average diameter, a minimum or maximum diameter. Such measurement may be repeated for each pattern, in particular each light wavelength, that is imaged. The measurements, such as diameters, may be visualized on the display 13.

The patterns are advantageously of a light wavelength in the visible range. One advantage is that the operator immediately receives feedback on whether the pattern is positioned on the desired target structure to be measured. Rather than positioning measurement points with an indicator on a display 13, the operator positions the pattern directly on the target structure by positioning the probe 11.

It may be possible to project light patterns of different wavelengths successively. The controller may be configured to start with a first light pattern, e.g. corresponding to a longest or shortest light wavelength, and sequentially changing the light wavelength until a measurement having a desirable accuracy is obtained. By way of example, when the target structure is small, projecting a light pattern of longer wavelength (e.g. red colour) may result in a pattern that does not interfere with the target structure (because it is too large). In such case, the controller may be configured to change the projected light wavelength which alters the size (and colour) of the projected light pattern. This may be implemented either automatically, e.g. the controller shifts wavelength when no measurement can be detected, or manually, e.g. with a push button allowing the operator to change projected wavelength. In these cases a light pattern corresponding to one light wavelength may be projected at a time.

By way of example, a diameter measurement of a bronchus is made preferably using a circular pattern. The pattern is positioned to fit the lumen and an image of the pattern is recorded and fed to the controller. The pattern can be automatically reconstructed in 3D, and the diameter determined according to known techniques. In case the probe is not centred in the bronchus lumen, the 3D reconstruction of the pattern will be tilted with respect to the optical axis. This tilt can be detected by the controller and which may be configured to emit a warning signal to the user/operator or to automatically correct the measurement. Another possibility is to combine several of the different patterns. In this case, the 3D reconstruction of the different patterns provides additional information of the lumen and may provide all the required data to get a reliable diameter measurement. In case of non-circular anatomical shapes, several measurements may be obtained through the pattern (reconstruction), all of which may be displayed on the visual display, such as the surface, the maximum diameter, the minimum diameter or any geometrical data that could be obtained.

When the contours of the structure that is to be measured is irregular, e.g. due to malformation, geometric quantities such as smallest circumscribed circle, and largest enclosed circle can be determined by controller 12 based on the automatically recognised/reconstructed pattern.

Controller 12 is therefore advantageously equipped with suitable software and/or hardware allowing for automatically recognising the pattern and/or measurement points on the pattern. In addition, the controller 12 is advantageously equipped with suitable software allowing for automatically determining a measurement from the automatically recognised pattern and/or measurement points. The measurement is advantageously a value representative of a diameter, perimeter, distance, or area. The pattern advantageously has a circular shape, e.g. a full circle, or line segments or points located on a circle. Such shape advantageously allows easy positioning of the pattern by the operator on the location where measurement is desired, and also allows easy automatic recognition and/or measurement calculation.

Advantageously, the controller is configured to perform sequential measurements, either with the same pattern, or with the separate patterns of different colours. Taking sequential measurements at e.g. regular time intervals with a same pattern may be useful for profiling of an internal lumen, or for averaging. Since the patterns of different colours have different size, taking sequential measurements with these patterns at a same target size may be useful for averaging, or for measuring different structures of the target size, e.g. at a constriction, the largest pattern may measure the size of the unobstructed aperture, while the smallest pattern may measure the size of the obstruction or of the obstructed aperture.

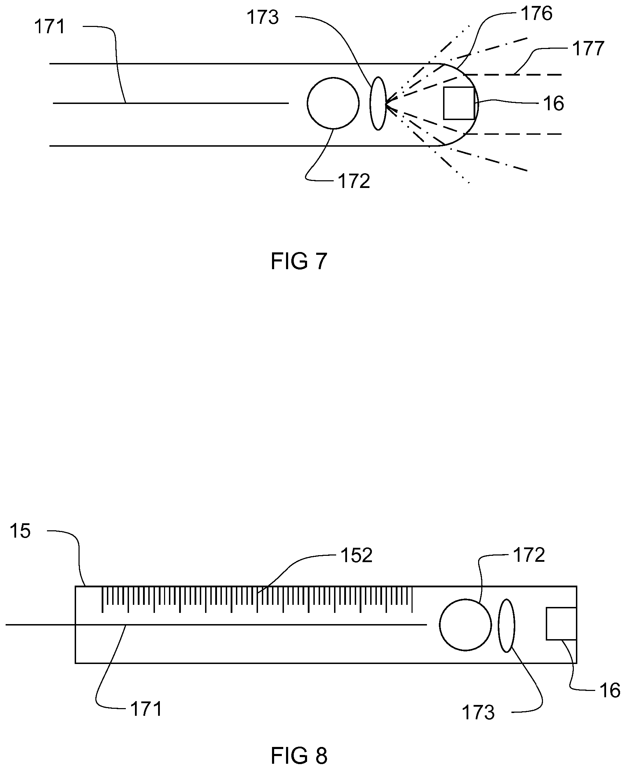

Advantageously, the light pattern projector 17 may comprise additional optical elements, in particular light refractors, in order to take advantage of the refraction to adjust the direction of the light pattern. Referring to Fig. 7, a light refractor 176 may be arranged downstream of the DOE 173. The light beam is first diffracted using the DOE 173 and the beam is refracted in another advantageous direction. Both combined give the projection angle. Using light refraction enables to have a final smaller projection angle and thus measure smaller elements. In Fig. 7, the shape is optimised in order to generate a telecentric pattern 177 (constant size along depth) in order to measure smaller elements.

In addition to any of the above, and referring to Fig. 8, the probe may comprise a graduated indication 152 on an outer surface of the housing 15. The indication 152 may be arranged along the longitudinal axis 151 of housing 15 and enable the endoscopist to determine how deep the probe has been inserted. This may enable to analyse e.g. how long a stenosis is. Measurement could be made using another endoscope (e.g. if the device passes through the instrument channel) or directly outside the patient as the user moves the probe in the patient.

The indication 152 can be an electronic encoder (e.g., optical encoder), to measure automatically the travel distance of housing 15. The encoder readings can be made in registration with the measurements based on the light pattern (diameter, area, perimeter). This can be useful, e.g. for determining the distance over which a contraction in a lumen extends. To this end, the electronic encoder can have an output coupled to controller 12 for reading travel distance in registration with performing the measurement(s).

Advantageously, the light pattern projector 17 is configured to generate a light pattern, e.g. one or more lines, possibly each in a different colour due to the use of coherent light beams of different colours, and one or more fiducial elements of light. One or more fiducial elements may be generated for each of the different colours.

The fiducial element of light is configured to form a fiducial marker on the surface of target site. The fiducial marker is advantageously shaped such that it is discernible by the operator, e.g. on the visual display 13 coupled to the controller 12. The operator manipulating the scoping instrument 9 can thus position the fiducial marker on a location of interest on the target site 8 merely by appropriate manipulation of the scoping instrument 9.

In some cases, it may be useful to measure entire 3D surfaces instead of only determining a distance measurement, e.g. between two anatomical points. In this case, multiple light sources are advantageously switched on successively. This enables e.g. to scan along the lumen. Additionally, some of these light sources can be switched on together too. In this case, advantageously more than three light sources are used.

The device 10 can further comprise an illumination light source. The illumination light source may be a source of white light or at least broadband light, which may not be coherent. The illumination light source can be optically coupled to the DOE 173 such that light emitted from the illumination light source passes through the DOE. Another possibility is to use a coherent light source that is not optically coupled to the DOE, such that its light is not diffracted using the DOE to provide a homogeneous illumination. Preferably, the illumination light source emits in a wavelength band different from the light wavelength of the light source 18 used for generating the pattern(s). The illumination light source and the pattern may be captured by different channels of the camera. By way of example, if the light source 18 is projecting a red pattern, the illumination light source can be blue and green. The illumination light source may be configured to emit in a plurality of selectable wavelengths.

A protecting sheath can be placed on the probe 11 in order to isolate the patient and any reusable part, such as the housing 15. This allows the probe to be easily reused with no contamination hazard. Additionally, the sheath can allow for preserving the optical properties of the probe, e.g. against scratching, as well as damages related to sterilisation.

Referring to Fig. 9, in case the conventional endoscopic system has no or a too small instrument channel, an external instrument channel 91 can be placed on the endoscope, through which the probe 11 can be moved. This external instrument channel may be attached to the endoscope through a sheath or a plastic or silicone ring 92.

**Claims**

1. Device (10) for non-contact measurement, comprising:

   a light source (18), a light pattern projector (17), an imaging system (16) configured for imaging a target site (8) illuminated by the light pattern projector,
   a support (15) to which the light pattern projector and the imaging system are attached in fixed relative positions, and
   a processing unit (12) configured to process data acquired by the imaging system,
   wherein the support has a longitudinal axis (151) parallel to an optical axis (175) of the light pattern projector (17), wherein the light pattern projector (17) and the imaging system (16) are arranged at spaced apart positions along the longitudinal axis (151),

   **characterised in that**:

   the light pattern projector (17) comprises a diffractive optical element (173) optically coupled to the light source (18),
   **in that**
   the light source is operable to emit a plurality of light beams of different colours, each one of the plurality of light beams being a coherent beam and being optically coupled to the diffractive optical element (173), such that the diffractive optical element is configured to diffract the plurality of light beams according to different diffraction angles resulting in separate patterns (201, 202, 203),
   **in that** the processing unit is configured to control the light source and/or the light pattern projector such that a single one of the separate patterns is projected at an instant of time, and/or **in that** the device comprises a user interface (122) configured to select a single one of the separate patterns for projection, and
   **in that** the processing unit is configured to determine a measurement based on at least two positions automatically recognised in data acquired from a single one of the separate patterns.

2. Device of claim 1, wherein the measurement is one or more of: a distance and an area.

3. Device of claim 2, wherein the distance is one of: diameter and perimeter.

4. Device of any one of the preceding claims, comprising an optical fibre (171) optically coupled between the light source and the diffractive optical element, wherein the optical fibre (171) is a single optical fibre, and wherein the light source is configured to send the plurality of light beams through the single optical fibre.

5. Device of any one of the preceding claims, wherein the light source comprises a beam combiner (186) configured to combine the plurality of light beams onto the optical fibre (171).

6. Device of any one of the preceding claims, wherein the plurality of light beams comprise a blue beam, a green beam and a red beam.

7. Device of any one of the preceding claims, wherein the processing unit (12) is configured to automatically determine the measurement based on the positions.

8. Device of any one of the preceding claims, wherein the processing unit (12) is configured to determine for a same target site and for each of the separate patterns positions of two or more points.

9. Device of any one of the preceding claims, further comprising a visual display (13), wherein the processing unit (12) is configured to indicate on the visual display the measurement.

**10.** Device of any one of the preceding claims, wherein the diffractive optical element (173) and the imaging system (16) are positioned to have parallel optical axes, wherein the imaging system is positioned in front of the diffractive optical element when considered in a direction of emission of light.

**11.** Device of any one of the preceding claims, wherein the light pattern projector (17) is configured to create concentric patterns.

**12.** Device of any one of the preceding claims, wherein the light pattern projector (17) is configured to project the separate patterns (201, 202, 203) having rotational symmetry about the optical axis (175), such as a circle, a square, or two parallel lines.

**13.** Device of any one of the preceding claim, wherein each of the separate patterns comprises a plurality of points arranged in a circle, or wherein each of the separate patterns consists of a circle.

**14.** Device of any one of the preceding claims, comprising a substantially cylindrical probe (11) with dimensions enabling the probe to travel along a channel of an endoscope (9), wherein the diffractive optical element (173) and the imaging system (16) are arranged in the probe.

**15.** Device of any one of the preceding claims, comprising an encoder operably coupled to the processing unit (12), wherein the encoder is configured to measure a travel distance of the support (15), preferably wherein the processing unit (12) is configured to determine the measurement in registration with a measurement of the travel distance.


**Patentansprüche**

**1.** Vorrichtung (10) zum kontaktlosen Messen, Folgendes umfassend:

eine Lichtquelle (18), einen Lichtmusterprojektor (17),
ein Bildgebungssystem (16), das dafür konfiguriert ist, eine Zielstelle (8) abzubilden, die durch den Lichtmusterprojektor angestrahlt wird,
einen Träger (15), an dem der Lichtmusterprojektor und das Bildgebungssystem in festen relativen Positionen angebracht sind, und
eine Verarbeitungseinheit (12), die dafür konfiguriert ist, Daten zu verarbeiten, die durch das Bildgebungssystem erfasst werden,
wobei der Träger eine Längsachse (151) aufweist, die parallel zu einer optischen Achse (175) des Lichtmusterprojektors (17) liegt, wobei der Lichtmusterprojektor (17) und das Bildgebungssystem (16) entlang der Längsachse (151) an voneinander beabstandeten Positionen angeordnet sind,
**dadurch gekennzeichnet, dass**:

der Lichtmusterprojektor (17) ein diffraktives optisches Element (173) umfasst, das optisch mit der Lichtquelle (18) gekoppelt ist,
dass
die Lichtquelle funktionsfähig ist, eine Vielzahl von Lichtstrahlen unterschiedlicher Farbe zu emittieren, wobei jeder der Vielzahl von Lichtstrahlen ein kohärenter Strahl ist und optisch mit dem diffraktiven optischen Element (173) gekoppelt ist, so dass das diffraktive optische Element dafür konfiguriert ist, die Vielzahl von Lichtstrahlen gemäß unterschiedlichen Beugungswinkeln zu beugen, die separate Mustern (201, 202, 203) ergeben,
dass die Verarbeitungseinheit dafür konfiguriert ist, die Lichtquelle und/oder den Lichtmusterprojektor derart zu steuern, dass in einem Moment ein einziges der separaten Muster projiziert wird, und/oder dass die Vorrichtung eine Benutzerschnittstelle (122) umfasst, die dafür konfiguriert ist, ein einziges der separaten Muster für das Projizieren auszuwählen, und
dass die Verarbeitungseinheit dafür konfiguriert ist, basierend auf mindestens zwei Positionen, die automatisch in von einem einzigen der separaten Muster erfassten Daten erkannt werden, einen Messwert zu bestimmen.

**2.** Vorrichtung nach Anspruch 1, wobei der Messwert eine Distanz und/oder ein Bereich ist.

**3.** Vorrichtung nach Anspruch 2, wobei die Distanz ein Durchmesser oder ein Außenumfang ist.

**4.** Vorrichtung nach irgendeinem der vorhergehenden Ansprüche, einen Lichtwellenleiter (171) umfassend, der optisch zwischen die Lichtquelle und das diffraktive optische Element gekoppelt ist, wobei der Lichtwellenleiter (171) ein einzelner Lichtwellenleiter ist und wobei die Lichtquelle dafür konfiguriert ist, die Vielzahl von Lichtstrahlen durch den einzelnen Lichtwellenleiter zu senden.

**5.** Vorrichtung nach irgendeinem der vorhergehenden Ansprüche, wobei die Lichtquelle einen Strahlkombinierer (186) umfasst, der dafür konfiguriert ist, die Vielzahl von Lichtstrahlen auf den Lichtwellenleiter (171) zu kombinieren.

**6.** Vorrichtung nach irgendeinem der vorhergehenden Ansprüche, wobei die Vielzahl von Lichtstrahlen einen blauen Strahl, einen grünen Strahl und einen roten Strahl umfassen.

**7.** Vorrichtung nach irgendeinem der vorhergehenden Ansprüche, wobei die Verarbeitungseinheit (12) dafür konfiguriert ist, den Messwert basierend auf den Positionen automatisch zu bestimmen.

**8.** Vorrichtung nach irgendeinem der vorhergehenden Ansprüche, wobei die Verarbeitungseinheit (12) dafür konfiguriert ist, für eine gleiche Zielstelle und für jedes der separaten Muster Positionen von zwei oder mehr Punkten zu bestimmen.

**9.** Vorrichtung nach irgendeinem der vorhergehenden Ansprüche, ferner eine visuelle Anzeige (13) umfassend, wobei die Verarbeitungseinheit (12) dafür konfiguriert ist, den Messwert auf der visuellen Anzeige anzugeben.

**10.** Vorrichtung nach irgendeinem der vorhergehenden Ansprüche, wobei das diffraktive optische Element (173) und das Bildgebungssystem (16) derart positioniert sind, dass sie parallele optische Achsen aufweisen, wobei das Bildgebungssystem, in Richtung der Lichtemission betrachtet, vor dem diffraktiven optischen Element (173) positioniert ist.

**11.** Vorrichtung nach irgendeinem der vorhergehenden Ansprüche, wobei der Lichtmusterprojektor (17) dafür konfiguriert ist, konzentrische Muster zu erzeugen.

**12.** Vorrichtung nach irgendeinem der vorhergehenden Ansprüche, wobei der Lichtmusterprojektor (17) dafür konfiguriert ist, die separaten Muster (201, 202, 203) mit Drehsymmetrie um die optische Achse (175) zu projizieren, wie beispielsweise als Kreis, als Quadrat oder als zwei parallele Linien.

**13.** Vorrichtung nach irgendeinem der vorhergehenden Ansprüche, wobei jedes der separaten Muster eine Vielzahl von Punkten umfasst, die in einem Kreis angeordnet sind, oder wobei jedes der separaten Muster aus einem Kreis besteht.

**14.** Vorrichtung nach irgendeinem der vorhergehenden Ansprüche, umfassend eine im Wesentlichen zylinderförmige Sonde (11) mit Abmessungen, welche die Sonde in die Lage versetzen, sich entlang eines Kanals eines Endoskops (9) zu bewegen, wobei das diffraktive optische Element (173) und das Bildgebungssystem (16) in der Sonde angeordnet sind.

**15.** Vorrichtung nach irgendeinem der vorhergehenden Ansprüche, einen Codierer umfassend, der funktionsfähig mit der Verarbeitungseinheit (12) gekoppelt ist, wobei der Codierer dafür konfiguriert ist, eine Bewegungsdistanz des Trägers (15) zu messen, vorzugsweise wobei die Verarbeitungseinheit (12) dafür konfiguriert ist, den Messwert in Deckung mit einem Messwert der Bewegungsdistanz zu bestimmen.

**Revendications**

**1.** Dispositif (10) pour mesure sans contact, comprenant :

une source de lumière (18), un projecteur de motif lumineux (17),
un système d'imagerie (16) configuré pour la prise d'images d'un site cible (8) éclairé par le projecteur de motif lumineux,
un support (15) auquel le projecteur de motif lumineux et le système d'imagerie sont fixés dans des positions relatives fixes, et
une unité de traitement (12) configurée pour traiter des données acquises par le système d'imagerie,
dans lequel le support a un axe longitudinal (151) parallèle à un axe optique (175) du projecteur de motif lumineux (17), dans lequel le projecteur de motif lumineux (17) et le système d'imagerie (16) sont agencés à des positions espacées le long de l'axe longitudinal (151),
**caractérisé en ce que** :

le projecteur de motif lumineux (17) comprend un élément optique diffractif (173) couplé optiquement à la source de lumière (18),
**en ce que**
la source de lumière permet d'émettre une pluralité de faisceaux lumineux de différentes couleurs, chacun de la pluralité de faisceaux lumineux étant un faisceau cohérent et étant couplé optiquement à l'élément optique diffractif (173), de telle sorte que l'élément optique diffractif est configuré pour diffracter la pluralité de faisceaux lumineux selon des angles de diffraction différents aboutissant à des motifs distincts (201, 202, 203),
**en ce que** l'unité de traitement est configurée pour commander la source de lumière et/ou le projecteur de motif lumineux de telle sorte qu'un seul des motifs distincts est projeté à un instant, et/ou **en ce que** le dispositif comprend une interface utilisateur (122) configurée pour sélectionner un seul des motifs distincts pour projection, et
**en ce que** l'unité de traitement est configurée pour déterminer une mesure sur la base d'au moins deux positions reconnues automatiquement dans des données acquises à partir d'un seul des motifs distincts.

2. Dispositif selon la revendication 1, dans lequel la mesure est une ou plusieurs d'une distance et d'une surface.

3. Dispositif selon la revendication 2, dans lequel la distance est l'un d'un diamètre et d'un périmètre.

4. Dispositif selon l'une quelconque des revendications précédentes, comprenant une fibre optique (171) couplée optiquement entre la source de lumière et l'élément optique diffractif, dans lequel la fibre optique (171) est une fibre optique unique, et dans lequel la source de lumière est configurée pour envoyer la pluralité de faisceaux lumineux à travers la fibre optique unique.

5. Dispositif selon l'une quelconque des revendications précédentes, dans lequel la source de lumière comprend un combinateur de faisceaux (186) configuré pour combiner la pluralité de faisceaux lumineux sur la fibre optique (171).

6. Dispositif selon l'une quelconque des revendications précédentes, dans lequel la pluralité de faisceaux lumineux comprend un faisceau bleu, un faisceau vert et un faisceau rouge.

7. Dispositif selon l'une quelconque des revendications précédentes, dans lequel l'unité de traitement (12) est configurée pour déterminer automatiquement la mesure sur la base des positions.

8. Dispositif selon l'une quelconque des revendications précédentes, dans lequel l'unité de traitement (12) est configurée pour déterminer pour un même site cible et pour chacun des motifs distincts des positions de deux points ou plus.

9. Dispositif selon l'une quelconque des revendications précédentes, comprenant en outre un affichage visuel (13), dans lequel l'unité de traitement (12) est configurée pour indiquer la mesure sur l'affichage visuel.

10. Dispositif selon l'une quelconque des revendications précédentes, dans lequel l'élément optique diffractif (173) et le système d'imagerie (16) sont positionnés de manière à avoir des axes optiques parallèles, dans lequel le système d'imagerie est positionné devant l'élément optique diffractif lorsqu'il est considéré dans une direction d'émission de lumière.

11. Dispositif selon l'une quelconque des revendications précédentes, dans lequel le projecteur de motif lumineux (17) est configuré pour créer des motifs concentriques.

12. Dispositif selon l'une quelconque des revendications précédentes, dans lequel le projecteur de motif lumineux (17) est configuré pour projeter les motifs distincts (201, 202, 203) ayant une symétrie rotationnelle autour de l'axe optique (175), tels qu'un cercle, un carré ou deux lignes parallèles.

13. Dispositif selon l'une quelconque des revendications précédentes, dans lequel chacun des motifs distincts comprend une pluralité de points agencés en un cercle, ou dans lequel chacun des motifs distincts consiste en un cercle.

14. Dispositif selon l'une quelconque des revendications précédentes, comprenant une sonde essentiellement cylindrique (11) avec des dimensions permettant à la sonde de se déplacer le long d'un canal d'un endoscope (9), dans lequel l'élément optique diffractif (173) et le système d'imagerie (16) sont agencés dans la sonde.

15. Dispositif selon l'une quelconque des revendications précédentes, comprenant un codeur couplé fonctionnellement à l'unité de traitement (12), dans lequel le codeur est configuré pour mesurer une distance de déplacement du support (15), de préférence dans lequel l'unité de traitement (12) est configurée pour déterminer la mesure en correspondance avec une mesure de la distance de déplacement.

10

11

111

14

9

122

121

12

13

FIG 1

112

174

15

151,
161, 175

16

171

172

173

17

FIG 2

FIG 3

FIG 4

FIG 5

FIG 6

FIG 7

FIG 8

91    11

9    92

FIG 9

203

202

201

200

FIG 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120190923 A
- WO 2017125926 A
- US 20160143509 A
- US 3971065 A, Bayer